(19) Europäisches Patentamt
European Patent Office
Office européen des brevets

(11) EP 0 530 657 B1

(12) **EUROPÄISCHE PATENTSCHRIFT**

(45) Veröffentlichungstag und Bekanntmachung des Hinweises auf die Patenterteilung:
**06.11.1996 Patentblatt 1996/45**

(51) Int Cl.6: **C07D 417/04**, C07B 57/00

(21) Anmeldenummer: **92114505.8**

(22) Anmeldetag: **26.08.1992**

(54) **Verfahren zur Enantiomerentrennung von 5-Hetaryl-1,3,4-thiadiazinon**

Process for the separation of enantiomers of 5-hetaryl-1,3,4-thiadiazinone

Procédé de séparation d'énantiomères de 5-hétaryl-1,3,4-thiadiazinone

(84) Benannte Vertragsstaaten:
**AT BE CH DE DK ES FR GB GR IE IT LI LU NL PT SE**

(30) Priorität: **02.09.1991 DE 4129062**

(43) Veröffentlichungstag der Anmeldung:
**10.03.1993 Patentblatt 1993/10**

(73) Patentinhaber: **MERCK PATENT GmbH**
**D-64271 Darmstadt (DE)**

(72) Erfinder:
- **Jonas, Rochus, Dr.**
  **W-6100 Darmstadt (DE)**
- **Ersing, Peter**
  **W-8751 Stockstadt a.M. (DE)**

(56) Entgegenhaltungen:
**EP-A- 0 225 503** **EP-A- 0 257 920**
**EP-A- 0 294 647**

- **ANGEWANDTE CHEMIE, Bd. 96, Nr. 2, 1984, WEINHEIM, DE Seiten 166 - 167 H. G[RTNER ET AL. 'Optisch aktive alpha-Arylcarbons uren durch kinetische Enantiomerentrennung: Pyrethroids uren'**
- **CHEMICAL ABSTRACTS, vol. 68, no. 25, 17. Juni 1968, Columbus, Ohio, US; abstract no. 113731m, H.J. BESTMANN ET AL. 'Reactions with alkylidenephosphoranes. XVIII. Kinetic racemate cleavage by reaction of alkylidenephosphoranes with optically active acid chlorides' Seite 10950 ;**

Bemerkungen:
Die Akte enthält technische Angaben, die nach dem Eingang der Anmeldung eingereicht wurden und die nicht in dieser Patentschrift enthalten sind.

EP 0 530 657 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein Verfahren zur Enantiomerentrennung von 5-Hetaryl-1,3,4-thiadiazinonen der Formel I

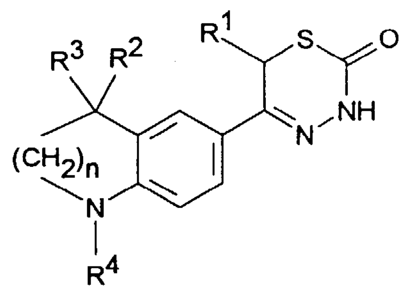

I

worin

| | |
|---|---|
| $R^1$ | A, |
| $R^2$ und $R^3$ | jeweils H oder A, |
| $R^4$ | H, A oder Acyl mit 1-15 C-Atomen, |
| A | Methyl, Ethyl, Propyl, Isopropyl, Butyl, Isobutyl, sek.-Butyl oder tert.-Butyl |

und

n 2 oder 3

bedeuten.

Thiadiazinonderivate der Formel I sind aus EP 0 294 647 bekannt und besitzen die dort bevorzugt angegebenen Bedeutungen. Kinetische Racematspaltungen anderer Verbindungen sind u.a. beschrieben in Angew. Chem., 1984, 96 (2), 166-7, wo gefunden wurde, daß optisch aktive α-Arylcarbonsäuren und deren Ester zu gewinnen sind, wenn man die Diastereomerengemische der α-Arylcarbonsäure-anhydride mit optisch aktivem 1-(3-Pyridyl)- oder 1-(4-Pyridyl)ethanol umsetzt.

In Chem. Abs., 1968, 68:113731m sind Racematspaltungen durch Reaktion von Alkylidenphosphoranen mit optisch aktiven Säurechloriden beschrieben, in EP-A-0225503 der Zusatz eines polymergebundenen Kristallisationsinhibitors und in EP-A-0257920 die selektive und unabhängige Kristallisation der Enantiomeren.

Vor- und nachstehend haben $R^1$ bis $R^4$ und A die bei der Formel I angegebene Bedeutung, wenn nicht ausdrücklich etwas anderes angegeben ist.

In den Formeln ist Alkyl vorzugsweise unverzweigt, hat vorzugsweise 1, 2, oder 3 C-Atome und steht bevorzugt für Methyl, ferner bevorzugt für Ethyl oder Propyl, weiterhin bevorzugt für Isopropyl, Butyl, Isobutyl, sek.-Butyl oder tert.-Butyl.

Acyl bedeutet den Säurerest einer Carbon- oder Sulfonsäure, vorzugsweise Alkanoyl mit 1-10, insbesondere 1, 2, 3, 4 oder 5 C-Atomen, im einzelnen bevorzugt Acetyl, ferner bevorzugt Formyl, Propionyl, Butyryl, Isobutyryl, Valeryl, Isovaleryl, Pivaloyl (Trimethylacetyl), weiterhin bevorzugt gegebenenfalls substituiertes Aroyl mit 7-15 C-Atomen, wobei als Substituenten insbesondere 1-3, vorzugsweise eine der folgenden Gruppen in Frage kommen:
Alkyl, Alkoxy, Alkylthio, Alkylsulfinyl oder Alkylsulfonyl mit jeweils 1-3, vorzugsweise 1 oder 2 C-Atomen, Methylendioxy, ferner OH, F, Cl, Br, J, $NO_2$, $NH_2$, Alkylamino oder Dialkylamin mit jeweils 1-3, vorzugsweise 1 oder 2 C-Atomen in der Alkylgruppe. Einzelne bevorzugte Aroylreste sind Benzoyl, o-, m- oder p-Toluyl, o-, m- oder p-Methoxybenzoyl, 2,3-, 2,4-, 2,5-, 2,6-, 3,4- oder 3,5-Dimethoxybenzoyl, 2,3,4-, 2,3,5-, 2,3,6-, 2,4,5-, 2,4,6- oder 3,4,5-Trimethoxybenzoyl, o-, m- oder p-Methylthiobenzoyl, o-, m- oder p-Methylsulfinylbenzoyl, o-, m- oder p-Methylsulfonylbenzoyl, 2,3- oder 3,4-Methylendioxybenzoyl, 1- oder 2-Napththoyl. Acyl kann weiterhin für Heteroarylcarbonyl mit 2-10 C-Atomen wie 2- oder 3-Furoyl, 2- oder 3-Thenoyl, Picolinoyl, Nicotinoyl, Isonicotinoyl stehen, außerdem für Arylalkanoyl wie Phenylacetyl, o-, m- oder p-Methoxyphenylacetyl, 2- oder 3-Phenylpropionyl, 2-, 3- oder 4-Phenylbutyryl; für Cycloalkylcarbonyl wie Cyclohexylcarbonyl; für Alkylsulfonyl wie Methyl-, Ethyl-, Propyl- oder Butylsulfonyl; für Arylsulfonyl wie Benzolsulfonyl, o-, m- oder p-Toluolsulfonyl, o-, m- oder p-Methoxybenzolsulfonyl, 1- oder 2-Naphthalinsulfonyl.

Die Trennung des Racemats in die jeweiligen Enantiomeren gelingt bisher nur durch aufwendige HPLC-Verfahren.

Der Erfindung lag die Aufgabe zugrunde, ein Verfahren zur Enantiomerentrennung von I zur Verfügung zu stellen, welches eine aufwendige HPLC-Trennung mit geringem Substanzdurchsatz umgeht, aber gleichzeitig hohe Enantiomerenreinheit in zufriedenstellenden Substanzmengen liefert.

Diese Aufgabe wurde durch die Auffindung des vorliegenden Verfahrens, der kinetischen Racematspaltung, im Hinblick auf die Enantiomerentrennung bei 5-Hetaryl-1,3,4-thiadiazinonen, gelöst.

Gegenstand der Erfindung ist demnach ein Verfahren zur Enantiomerentrennung von I, dadurch gekennzeichnet, daß man racemisches I in einem inerten Lösungsmittel oder Lösungsmittelgemisch löst, mit einem chiralen Säurechlorid acyliert, das entstandene Diastereomerengemisch mit einem Amin oder Alkohol umsetzt, dabei eine vollstandige Spaltung des einen Diastereomeren und eine geringfügige Spaltung des anderen Diasteromeren zu den zugrundeliegenden Enantiomeren erreicht, die Spaltprodukte anschließend abtrennt und das verbleibende reine Diastereomere durch Reaktion mit einem Amin oder einem Alkohol in das entsprechende reine Enantiomere überführt.

Das Verfahren der kinetischen Racematspaltung liefert in der Regel keine zufriedenstellende Enantiomerenreinheit und muß durch zusätzliche Verfahren ergänzt werden.

Daher ist es überraschend, daß es im Falle von I mit Erfolg angewendet werden kann und ohne den Einsatz von ergänzenden Methoden in allen Beispielen eine Enantiomerenreinheit von mehr als 99 % liefert.

Als Lösungsmittel eignen sich vorzugsweise Ether wie Tetrahydrofuran (THF), Dioxan oder Methyl-tert.-butylether, Kohlenwasserstoffe wie Hexan, Cyclohexan, Benzol, Toluol, Xylole oder Mesitylen, Glykoldialkylether wie Glykoldimethyl- oder -diethylether, Amide wie Diemthylformamid (DMF), halogenierte Kohlenwasserstoffe wie Dichlormethan, Chlorbenzol oder Trichlorethylen sowie Gemische dieser Lösungsmittel.

Besonders bevorzugt sind Dichlormethan und THF.

Geeignete optisch-aktive Säurechloride sind z.B. Tetrahydro-5-oxo-2-furancarbonsäurechlorid, o-Acetylmandelsäurechlorid, Campholsäurechlorid oder besonders bevorzugt, Camphansäurechlorid.

Im einzelnen löst oder suspendiert man racemisches I in einem der genannten Lösungsmittel bzw. einem Lösungsmittelgemisch, fügt zweckmäßigerweise eine Base hinzu und gibt das Säurechlorid, gelöst in einem der genannten Lösungsmittel oder in reiner Form dazu. Als Basen eignen sich z.B. Alkalimetall- oder Erdalkalimetallhydroxide, -carbonate, -alkoholate, insbesondere aber sekundäre oder tertiäre Amine wie z.B. Triethylamin oder Pyridin. Die Reaktionsmischung wird danach 1-48 Std. bei Temperaturen zwischen -20° und dem Siedepunkt des Lösungsmittels, vorzugsweise im Bereich von -10° bis +30° gerührt und das Diastereomerengemisch isoliert. Zur Spaltung der Diastereomerenmischung wird diese erneut in einem der genannten Lösungsmittel gelöst, mit einem Amin oder einem Alkohol versetzt und wiederum 1-48 Std. bei 0-50°, vorzugsweise bei 0-30° gerührt oder einfach stehengelassen.

Es ist ebenso möglich, die Diastereomerenmischung ohne Verwendung eines zusätzlichen Lösungsmittels direkt in einem geeigneten Alkohol zu lösen.

Geeignete Alkohole sind beispielsweise niedere Alkohole mit 1-8 C-Atomen, insbesondere Methanol, Ethanol oder Isopropanol, aber auch Gemische derselben. Geeignete Amine sind u.a. Piperidin, Pyrrolidin, Morpholin oder auch Ethylamin.

In den nachstehenden Beispielen, die dazu dienen die Erfindung näher zu erläutern, sind ebenso wie in dem vorhergehenden Text alle Temperaturen in °C angegeben. "Übliche Aufarbeitung" bedeutet, man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über $Na_2SO_4$ oder $MgSO_4$, filtriert, dampft ein und reinigt gegebenenfalls zusätzlich durch Chromatographie oder Kristallisation. Die Enantiomereneinheit kann beispielsweise durch HPLC oder "Differential Scanning Calorimetry" ("DSC") bestimmt werden. Die Abkürzungen "HPLC" und "ee" stehen für High pressure liquid chromatography" und für "enantiomeric excess".

## Beispiel 1

Zu einer Suspension von 48 g 5-[1-(3,4-Methylendioxybenzoyl)-1,2,3,4-tetrahydro-6-chinolyl]-6-methyl-3,6-dihydro-2H-1,3,4-thiadiazin-2-on in 800 ml Dichlormethan, versetzt mit 20 ml Triethylamin, tropft man unter Rühren bei 0° 26 g (-)-Camphansäurechlorid, gelöst in 100 ml Dichlormethan, hinzu und rührt 4 Std. Anschließend wird die Reaktionsmischung mit verdünnter Salzsäure und nachfolgend mit Bicarbonatlösung gewaschen. Die organische Phase wird abgetrennt und wie üblich aufgearbeitet. Man erhält 3-[(-)-Camphanoyl]-5-[1-(3,4-methylendioxybenzoyl)-1,2,3,4-tetrahydro-6-chinolyl]-6-methyl-3,6-dihydro-2H-1,3,4-thiadiazin-2-on als Diastereomerengemisch, F. 216-217°.

## Beispiel 2

48 g Diastereomerengemisch aus Beispiel 1 werden in 800 ml Tetrahydrofuran gelöst und nach Zugabe von 3,6

ml Morpholin 14 Std. bei 25° stehengelassen. Man engt die Reaktionsmischung ein, versetzt sie mit wässrigem Ethylacetat und arbeitet wie üblich auf. Das durch Spaltung entstandene (-)-Enantiomere, welches durch geringe Mengen (+)-Enantiomeres verunreinigt ist, wird von der Hauptmenge des nicht gespaltenen Diastereomeren durch Chromatographie abgetrennt. Das im Unterschuß vorliegende (+)-Enantiomere wird vom (-)-Enantiomeren durch Umkristallisation aus Ethanol als Racemat entfernt. Nach Einengen der Mutterlauge und Kristallisation erhält man (-) -5-[1-(3,4-Methylendioxybenzoyl)-1,2,3,4-tetrahyro-6-chinolyl]-6-methyl-3,6-dihydro-2H-1,3,4-thiadiazin-2-on, F. 180°; $[\alpha]_D^{20}$ = -534,2°; ee > 99 % (HPLC).

**Beispiel 3**

20 g des nicht gespaltenen Diastereomeren aus Beispiel 2 werden in THF gelöst, mit 3 ml Morpholin versetzt und analog zu Beispiel 2 weiterverarbeitet. Nach Entfernung des Lösungsmittels wird der Rückstand aus Ethanol umkristallisiert. Man erhält (+)-5-[1-(3,4-Methylendioxybenzoyl)-1,2,3,4-tetrahydro-6-chinolyl]-6-methyl-3, 6-dihydro-2H-1,3, 4-thiadiazin-2-on, F. 181° $[\alpha]_D^{20}$ = +541,5°; ee > 99 %.

**Beipiel 4**

20 g der nicht gespaltenen diastereomeren Verbindung aus Beispiel 2 werden in 400 ml Methanol gelöst und 24 Std. gekocht. Nach Entfernung des Lösungsmittels kristallisiert man den Rückstand aus Ethanol um und erhält (+)-5-[1-(3,4-Methylendioxybenzoyl)-1,2,3,4-tetrahydro-6-chinolyl]-6-methyl-3,6-dihydro-2H-1,3,4-thiadiazin-2-on, F. 181°; $[\alpha]_D^{20}$ = +541,5°; ee > 99 %.

**Beispiel 5**

Analog Beispiel 1 setzt man die racemische Mischung von 5-[1-Methyl-1,2,3,4-tetrahydro-6-chinolyl]-6-methyl-3, 6-dihydro-2H-1,3,4-thiadiazin-2-on (F. 177°) mit (+)-Camphansäurechlorid um. Man erhält 3-[(+)-Camphanoyl]-5-(1-methyl-1,2,3,4-tetrahydro-6-chinolyl)-6-methyl-3,6-dihydro-2H-1,3,4-thiadiazin-2-on als Diastereomerengemisch.

**Beispiel 6**

Das Diastereomerengemisch aus Beispiel 5 wird in Analogie zu Beispiel 2 mit Morpholin umgesetzt. Man erhält (+)-5-(1-Methyl-1,2,3,4-tetrahydro-6-chinolyl)-6-methyl-3,6-dihydro-2H-1,3,4-thiadiazin-2-on sowie das entsprechende nicht gespaltene Diastereomere, dessen Weiterverarbeitung in Beispiel 7 beschrieben ist.

**Beispiel 7**

Das nicht gespaltene Diastereomere aus Beispiel 6 wird analog zu Beispiel 4 in Methanol gelöst und 20 Std. gekocht. Nach Entfernung des Lösungsmittels kristallisiert man den Rückstand aus Ethanol um und erhält (-)-5-(1-Methyl-1,2,3,4-tetrahydro-6-chinolyl)-6-methyl-3,6-dihydro-2H-1,3,4-thiadiazin-2-on.

**Beispiel 8**

Analog Beispiel 1 setzt man das Racemat von 5-[1-(3,4,5-Trimethoxybenzoyl)-1,2,3,4-tetrahydro-6-chinolyl]-6-methyl-3,6-dihydro-2H-1,3,4-thiadiazin-2-on mit (-)-Camphansäurechlorid um. Man erhält 3-[(+)-Camphanoyl]-5-[1-(3,4,5-Trimethoxybenzoyl)-1,2,3,4-tetrahydro-6-chinolyl]-6-methyl-3,6-dihydro-2H-1,3,4-thiadiazin-2-on als Diastereomerengemisch.

Analog erhält man

3-[(+)-Camphanoyl]-5-[1-Isonicotinoyl-2,3,4,5-tetrahydro-1H-1-benzazepin-7-yl]-6-methyl-3,6-dihydro-2H-1,3,4-thiadiazin-2-on.

**Beispiel 9**

Das Diastereomerengemisch aus Beispiel 8 wird analog zu Beispiel 2 mit Morpholin umgesetzt. Man erhält (-)-5-[1-(3,4,5-Trimethoxybenzoyl)-1,2,3,4-tetrahydro-6-chinolyl]-6-methyl-3,6-dihydro-2H-1,3,4-thiadiazin-2-on sowie das entsprechende nicht gespaltene Diastereomere, dessen Weiterverarbeitung in Beispiel 10 beschrieben ist.

Analog erhält man

(-)-5-[1-Isonicotinoyl-2,3,4,5-tetrahydro-1H-1-benzazepin-7-yl]-6-methyl-3,6-dihydro-2H-1,3,4-thiadiazin-2-on.

**Beispiel 10**

Analog Beispiel 3 erhält man ausgehend von dem nicht gespaltenen Diastereomeren aus Beispiel 9 (+)-5-[1-(3,4,5-Trimethoxybenzoyl)-1,2,3,4-tetrahydro-6-chinolyl]-6-methyl-3,6-dihydro-2H-1,3,4-thiadiazin-2-on, $[\alpha]_D^{20}$ = +476,2°.

Analog erhält man

(+)-5-[1-Isonicotinoyl-2,3,4,5-tetrahydro-1H-1-benzazepin-7-yl]-6-methyl-3,6-dihydro-2H-1,3,4-thiadiazin-2-on, $[\alpha]_D^{20}$ = +478,2°.

## Patentansprüche

1. Verfahren zur Enantiomerentrennung von 5-Hetaryl-1,3,4-thiadiazinonen der Formel I

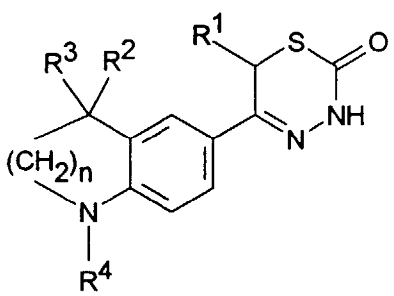

I

worin

| | |
|---|---|
| $R^1$ | A, |
| $R^2$ und $R^3$ | jeweils H oder A, |
| $R^4$ | H, A oder Acyl mit 1-15 C-Atomen, |
| A | Methyl, Ethyl, Propyl, Isopropyl, Butyl, Isobutyl, sek.-Butyl oder tert.-Butyl |

und

n 2 oder 3

bedeuten,
durch kinetische Racematspaltung, dadurch gekennzeichnet, daß man Racemate der Verbindungen der Formel I in einem inerten Lösungsmittel oder Lösungsmittelgemisch löst, mit (-)- oder (+)-Camphansäurechlorid acyliert, das entstandene Diastereomerengemisch mit einem sekundären oder tertiären Amin umsetzt, dabei eine vollständige Spaltung eines Diastereomeren und eine geringfügige Spaltung des anderen Diastereomeren zu den zugrundeliegenden Enantiomeren erreicht, die Spaltprodukte anschließend abtrennt und das verbleibende reine Diastereomere durch Reaktion mit einem Amin oder einem Alkohol in das entsprechend reine Enantiomere überführt.

2. Verfahren gemäß Anspruch 1, dadurch gekennzeichnet, daß man zur Diastereomerenspaltung Alkohole verwendet.

## Claims

1. A process for the resolution of enantiomers of 5-heteroaryl-1,3,4-thiadiazinones of formula I:

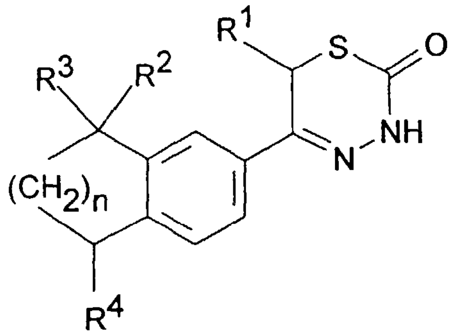

I

wherein

| | |
|---|---|
| $R^1$ | is A, |
| $R^2$ and $R^3$ | are each H or A, |
| $R^4$ | is H, A or acyl having 1-15 C atoms, |
| A | is methyl, ethyl, propyl, isopropyl, butyl, isobutyl, sec.-butyl or tert.-butyl |

and

n is 2 or 3,

by the kinetic resolution of racemates, characterised in that racemates of the compounds of formula I are dissolved in an inert solvent or solvent mixture and acylated with (-)- or (+)-camphanic acid chloride, the resulting mixture of diastereoisomers is reacted with a secondary or tertiary amine, thereby achieving a complete resolution of one of the diastereoisomers and a very slight resolution of the other diastereoisomer into the enantiomers on which they are based, the resolution products are then separated off and the remaining pure diastereoisomer is converted to the corresponding pure enantiomer by reaction with an amine or an alcohol.

2. A process according to Claim 1, characterised in that alcohols are used for resolving the diastereoisomers.

## Revendications

1. Procédé de séparation d'énantiomers de 5-hétéroaryle-1,3-thiadiazinones de formule I

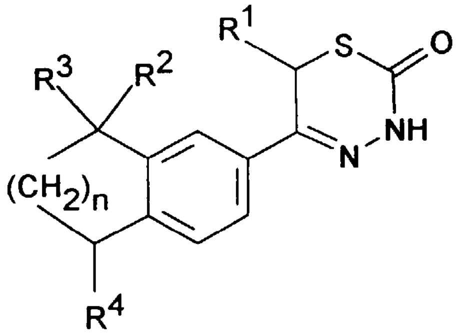

I

dans laquelle

$R^1$ représente A,

$R^2$ et $R^3$ représentent chacun H ou A,

$R^4$ représente H, A ou alkanoyle de 1 à 15 atomes de carbone,

A représente méthyle, éthyle, n-propyle, isopropyle, n-butyle, isobutyle, sec.-butyle ou tert.-butyle, et

n est égale à 2 ou 3

par séparation cinétique de racémates, caractérisé en ce que l'on dissoud des racémates des composés de formule I dans un solvant indifférent ou dans un mélange de solvants, les fait réagir avec la (-) - ou (+)- chlorure d'acide camphanique, que l'on fait réagir le mélange de diastéréomères obtenu avec une amines secondaire ou tertiaire, en obtenant une séparation complète d'un des diastéréomère et une séparation insignifiante de l'autre diastéréomère fournissant les énantiomères qui sont à la base, que l'on sépare enfin les produits de séparation et que l'on transforme le diastérémère pure qui reste en l'énantiomère pure correspondant par réaction avec une amine ou alcool.

2. Procédé selon la revendication 1, caractérisé en ce que l'on utilise des alcools pour séparer les diastéréomères